# EUROPEAN PATENT APPLICATION

(11) **EP 1 889 627 A1**
(43) Date of publication of application: **20.02.2008**
(21) Application number: 06747090.6
(22) Date of filing: 01.06.2006
(51) Int. Cl.: A61K 38/22, A61K 35/12, A61K 35/28, A61P 9/10

(54) **THERAPEUTIC AGENT FOR BLOOD-RELATED DISEASE CONTAINING EPO DERIVATIVE**

(30) Priority: 01.06.2005 JP 2005161005
(71) Applicant: Niigata TLO Corporation, Niigata-shi Niigata 950-2102 (JP)
(72) Inventor: TOBA, Ken, 9518113 (JP); KATO, Kiminori, 9500861 (JP); HIGUCHI, Masato, c/o Chugai Seiyaku K.K., Gotenba-shi, Shizuoka 412-8513 (JP)
(74) Representative: HOFFMANN EITLE
(86) International application number: PCT/JP2006/310989
(87) International publication number: WO 2006/129755

(57) **Abstract**

Blood flow enhancers, vascular promoters or therapeutic agents for ischemic diseases containing an erythropoietin (EPO) derivative as an active ingredient.

## Description

### TECHNICAL FIELD

The present invention relates to blood flow enhancers, vascular promoters or therapeutic agents for ischemic diseases containing an EPO derivative as an active ingredient.

### BACKGROUND ART

Erythropoietin (hereinafter sometimes referred to as EPO) is an acidic glycoprotein hormone which promotes the differentiation and proliferation of erythroid progenitor cells, and mainly produced by the kidney. The major blood components erythrocytes are destroyed in the spleen and the like after a certain period of function (a mean lifetime of about 120 days in humans), but constantly supplied from the bone marrow so that the total number of peripheral erythrocytes is always kept in a normal state. EPO plays a central role in maintaining erythrocyte homeostasis in vivo. EPO is clinically used for the treatment of anemia and pre-and post-operative management.

It has also been reported that EPO has an angiogenesis promoting effect and is useful as therapeutic agents for ischemic diseases (Anatole B et al., The New England Journal of Medicine, 339(9), 584-590, (1998), Christopher H et al., Blood, 102(4), 1340-1346, (2003), Ferdinand H B et al., Blood,103(3),921-926, (2004), Kyle J S et al., Cardiovascular Research, (59), 538-548, (2003), Ferdinand H B et al., Kidney International, 64, 1648-1652, (2003)). However, administration of EPO for the purpose of angiogenetic effect may cause an increase in erythrocytes contrary to the intended purpose. Thus, there is a need for novel therapeutic agents that can be used for blood-related diseases such as ischemic diseases while minimizing the increase in erythrocytes.

### DISCLOSURE OF THE INVENTION

### PROBLEMS TO BE SOLVED BY THE INVENTION

An object of the present invention is to provide a therapeutic agent that can be used for ischemic diseases and the like.

### MEANS TO SOLVE THE PROBLEMS

We accomplished the present invention on the basis of the finding that EPO derivatives, especially asialoEPO have a blood flow enhancing effect, vascular promoting effect or therapeutic effect for ischemic diseases.

Accordingly, the present invention provides the following.
(1) A therapeutic agent for ischemic diseases containing an erythropoietin (EPO) derivative as an active ingredient.
(2) The therapeutic agent as defined in (1) wherein the EPO derivative is asialoEPO.
(3) The therapeutic agent as defined in (1) or (2), which is administered in combination with a stem cell-containing material.
(4) The therapeutic agent as defined in (3) wherein the stem cell-containing material is bone marrow cell.
(5) A vascular promoter containing an EPO derivative as an active ingredient.
(6) The promoter as defined in (5) wherein the EPO derivative is asialoEPO.
(7) The promoter as defined in (5) or (6), which is administered in combination with a stem cell-containing material.
(8) The promoter as defined in (7) wherein the stem cell-containing material is bone marrow cell.
(9) A blood flow enhancer containing an EPO derivative as an active ingredient.
(10) The enhancer as defined in (9) wherein the EPO derivative is asialoEPO.
(11) The enhancer as defined in (9) or (10), which is administered in combination with a stem cell-containing material.
(12) The enhancer as defined in (11) wherein the stem cell-containing material is bone marrow cell.

### BRIEF EXPLANATION OF THE DRAWINGS

Figure 1 is a graph showing the results of a therapeutic experiment using a hindlimb ischemia model of ICR mice treated with an EPO derivative (left: survival effect, right: vascular regeneration effect).
Figure 2 is a graph showing the results of a therapeutic experiment using the hindlimb ischemia model of ICR mice treated with an EPO derivative in combination with bone marrow cells (left: survival effect, right: vascular regeneration effect).
Figure 3 is a graph showing the results of a therapeutic experiment using the hindlimb ischemia model of ICR mice (blood flow recovery effect).
Figure 4 is a graph showing the results of a therapeutic experiment using the hindlimb ischemia model of ICR mice (angiogenetic effect).
Figure 5 is a graph showing the effects of EPO and **asialoEPO on the development of polycythemia in untreated ICR** mice.

### THE MOST PREFERRED EMBODIMENTS OF THE INVENTION

### EPO

The EPO used in the present invention can be any EPO, but preferably highly purified EPO, more specifically mammalian EPO, especially having biological activity substantially identical to that of human EPO.

The EPO used in the present invention can be those prepared by any process, including e.g., natural human EPO purified from human-derived extracts (JPB HEI 1-38800, etc.), or human EPO recombinantly produced in E.coli, yeast cells, Chinese hamster ovary cells (CHO cells), C127 cells, COS cells, myeloma cells, BHK cells, insect cells or the like and extracted and isolated/purified by various methods. The EPO used in the present invention is preferably recombinantly prepared, preferably by using mammalian cells (especially CHO cells) (e.g., JPB HEI 1-44317, Kenneth Jacobs et al., Nature, 313 806-810 (1985), etc.).

Recombinantly obtained EPO may have an amino acid sequence identical to that of naturally derived EPO or may contain a deletion, substitution, addition or other modification of one or more amino acids in the amino acid sequence so far as it has similar biological activity to that of naturally derived EPO. Amino acid deletion, substitution, addition or other modification can be performed by methods known to those skilled in the art. For example, a polypeptide functionally comparable to EPO can be prepared by those skilled in the art by introducing an amino acid variation into EPO as appropriate via site-directed mutagenesis (Gotoh, T. et al. (1995) Gene 152, 271-275; Zoller, M.J. and Smith, M. (1983) Methods Enzymol. 100, 468-500; Kramer, W. et al. (1984) Nucleic Acids Res. 12, 9441-9456; Kramer, W. and Fritz, H.J. (1987) Methods Enzymol. 154, 350-367; Kunkel, T.A. (1985) Proc. Natl. Acad. Sci. USA. 82, 488-492; Kunkel (1988) Methods Enzymol. 85, 2763-2766) or other techniques. Amino acid variations also occur in nature. Generally, an amino acid residue is preferably substituted by another amino acid residue in which the property of the amino acid side chain is conserved. For example, the properties of amino acid side chains include hydrophobic amino acids (A, I, L, M, F, P, W, Y, V), hydrophilic amino acids (R, D, N, C, E, Q, G, H, K, S, T), amino acids having aliphatic side chains (G, A, V, L, I, P), amino acids having hydroxyl-containing side chains (S, T, Y), amino acids having sulfur-containing side chains (C, M), amino acids having carboxylate- and amide-containing side chains (D, N, E, Q), amino acids having base-containing side chains (R, K, H), and amino acids having aromatic-containing side chains (H, F, Y, W) (examples shown by one-letter amino acid codes within parentheses). It has been already known that polypeptides having an amino acid sequence modified by deleting, adding and/or substituting one or more amino acid residues retain their biological activity (Mark, D.F. et al., Proc. Natl. Acad. Sci. USA (1984) 81, 5662-5666; Zoller, M.J. & Smith, M. Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413).

Fusion proteins of EPO and another protein can also be used. Fusion proteins can be prepared by e.g. ligating the DNA encoding EPO in-frame with the DNA encoding another protein, inserting the ligation product into an expression vector and expressing it in a host. The second protein to be fused to EPO in the present invention is not specifically limited.

Chemically modified EPO can also be used. Examples of chemically modified EPO include, for example, EPO conjugated to a compound such as an inorganic or organic compound, e.g., polyethylene glycol, vitamin B12, etc.

### EPO derivatives

As used herein, EPO derivative refers to EPO containing a modified amino acid in the EPO molecule or to EPO containing a modified carbohydrate chain in the EPO molecule.

Modifications of carbohydrate chains in EPO molecules include addition, substitution, deletion and the like of carbohydrate chains. Preferred carbohydrate modifications in the present invention include deletion of sialic acids in EPO molecules.

Normally, both of EPO produced by recombinant animal cells and EPO derived from urine are obtained as EPO compositions containing various EPO molecules having different carbohydrate structures. The number of sialic acids attached to the EPO molecules in the EPO compositions depends on the specific EPO molecules, but normally 11 to 15 sialic acids are attached to one EPO molecule. Desialylated EPO (asialoEPO) can be prepared by removing these sialic acids. The number of sialic acids removed by desialylation is not specifically limited, and all sialic acids may be removed, or 1, 2, 3, 4, 5, 6, 7, 8, 9, 10, 11, 12, 13 or 14 sialic acids may be removed. Preferred asialoEPO in the present invention has 10 or less, more preferably 5 or less, most preferably 2 or less sialic acids attached to the EPO molecule. It should be noted that the number of sialic acids used in the present invention is the average number in EPO molecules contained in EPO compositions. The average number of sialic acids per molecule can be determined by methods known to those skilled in the art (EP0428267, etc.).

Desialylated EPO (asialoEPO) can be prepared by methods known to those skilled in the art, e.g., by treating EPO with an enzyme such as sialidase. Sialidases are commercially available (JPA 2005-507426, Nobuo Imai et al., Eur.J.Biochem, 194, 457-462 (1990), etc.).
Modifications of amino acids in EPO molecules include carbamylation, biotinylation, amidination, acetylation, guanidinylation, etc., but a preferred amino acid modification in the present invention is carbamylation.

Amino acid residues modified are not limited, including, e.g., lysine, arginine, glutamic acid, tryptophan, etc., but a preferred amino acid modified in the present invention is lysine.

Thus, an especially preferred embodiment of EPO containing a modified amino acid is EPO containing carbamylated lysine (Marcel L et al. Derivatives of erythropoietin that are tissue protective but not erythropoietic. Science, 2004; 305: 239, Fiordaliso E et al. A nonerythropoietic derivative of erythropoietin protects the myocardium from ischemia-reperfusion injury. PNAS, 2005; 102: 2046, etc.). Carbamylations of EPO include carbamylation mediated by reaction with cyanate ions or the like; alkyl carbamylation mediated by reaction with alkyl isocyanates or the like; aryl carbamylation mediated by reaction with aryl isocyanates or the like.

### Diseases

The therapeutic agents of the present invention are useful as blood flow enhancers, vascular promoters or therapeutic agents for ischemic diseases.

As used herein, blood flow enhancement means that the blood flow at a site to which an EPO derivative was topically administered or a distal site increases as compared with the blood flow before administration or that the blood flow reaching ischemic tissue increases. Whether or not blood flow enhancement has been achieved can be determined by methods known to those skilled in the art, e.g., by rheologically measuring blood flow using radioactive microspheres (Am. J. Physiol. 243:H371-H378, (1982)). Blood flow enhancement can also be measured/checked by skin temperature, thermography, laser blood flowmetry, lower limb/upper limb tension ratio, tissue oxygen partial pressure and the like.

As used herein, vascular promotion refers to promotion of angiogenesis, promotion of vascular regeneration, or promotion of growth/development of blood vessels. Blood vessels promoted to form, regenerate, grow or develop by EPO derivatives are not specifically limited, but preferably arteries, especially peripheral arteries. Whether or not vascular promotion has been achieved can be determined by methods known to those skilled in the art, e.g., by measuring capillary density using alkali phosphatase staining. It can also be determined/checked by angiography or the like.

The therapeutic agents of the present invention are useful for treating ischemic diseases. Ischemic diseases are conditions associated with decreased blood flow in vessels and ischemic tissue caused by various factors such as narrowed vascular lumen, clot formation, vascular occlusion, vasculitis, vascular contraction, increased blood viscosity, etc. Examples of ischemic diseases include peripheral circulation disorders, ischemic heart diseases (ischemic cardiomyopathy, myocardial infarction, ischemic heart failure, etc.), ischemic renal diseases, ischemic lung diseases, infection-related ischemic diseases, etc.

Ischemic diseases to be treated with the therapeutic agents for ischemic diseases of the present invention are not limited and they are applied to any ischemic disease, but preferably peripheral circulation disorders. Peripheral circulation disorders are conditions associated with decreased blood flow in peripheral arteries and ischemic peripheral tissue caused by narrowed vascular lumen, clot formation, vascular occlusion, vasculitis, vascular contraction, increased blood viscosity, etc. Diseases involving peripheral circulation disorders include arteriosclerosis obliterans, chronic arterial obstruction such as Buerger's disease, progressive systemic sclerosis, systemic lupus erythematosus, Raynaud's disease, vibration disease, aneurysm, vasculitis, etc.

### Therapeutic formulations

The therapeutic agents of the present invention can contain suspending agents, solubilizers, stabilizers, isotonizing agents, preservatives, adsorption inhibitors, surfactants, diluents, excipients, pH modifiers, soothing agents, buffers, sulfur-containing reducing agents, antioxidants, etc., as appropriate.

Examples of suspending agents include methylcellulose, Polysorbate 80, hydroxyethylcellulose, gum acacia, gum tragacanth powder, sodium carboxymethylcellulose, polyoxyethylene sorbitan monolaurate, etc.

Solubilizers include polyoxyethylene hydrogenated castor oil, Polysorbate 80, nicotinic acid amide, polyoxyethylene sorbitan monolaurate, Macrogols, castor oil fatty acid ethyl esters, etc.

Stabilizers include dextran 40, methylcellulose, gelatin, sodium sulfite, sodium metasulfite, etc.

Certain amino acids can also be included as stabilizers (e.g., JPA HEI 10-182481, etc.). Amino acids added as stabilizers include free amino acids and salts thereof such as sodium salts, potassium salts, hydrochlorides, etc. Amino acids can be added alone or as a combination of two or more. Amino acids added as stabilizers are not specifically limited, but preferred amino acids include leucine, tryptophan, serine, glutamic acid, arginine, histidine and lysine.

Isotonizing agents include e.g., D-mannitol, sorbitol, etc.

Preservatives include e.g., methyl paraoxybenzoate, ethyl paraoxybenzoate, sorbic acid, phenol, cresol, chlorocresol, etc.

Adsorption inhibitors include e.g., human serum albumin, lecithin, dextran, ethylene oxide/propylene oxide copolymers, hydroxypropylcellulose, methylcellulose, polyoxyethylene hydrogenated castor oil, polyethylene glycol, etc.

Typical examples of surfactants include:
nonionic surfactants, e.g., sorbitan fatty acid esters such as sorbitan monocaprylate, sorbitan monolaurate, sorbitan monopalmitate; glycerin fatty acid esters such as glycerin monocaprylate, glycerin monomyristate, glycerin monostearate; polyglycerin fatty acid esters such as decaglyceryl monostearate, decaglyceryl distearate, decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate; polyoxyethylene sorbitol fatty acid esters such as polyoxyethylene sorbitol tetrastearate, polyoxyethylene sorbitol tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether, polyoxyethylene polyoxypropylene cetyl ether; polyoxyethylene alkyl phenyl ethers such as polyoxyethylene nonyl phenyl ether; polyoxyethylene hardened castor oils such as polyoxyethylene castor oil, polyoxyethylene hardened castor oil (polyoxyethylene hydrogenated castor oil); polyoxyethylene beeswax derivatives such as polyoxyethylene sorbitol beeswax; polyoxyethylene lanolin derivatives such as polyoxyethylene lanolin; polyoxyethylene fatty acid amides such as polyoxyethylene stearic acid amide, each of which has an HLB of 6-18;
anionic surfactants, e.g., alkyl sulfates having a C10-18 alkyl group such as sodium cetyl sulfate, sodium lauryl sulfate, sodium oleyl sulfate; polyoxyethylene alkyl ether sulfates having an average EO mole number of 2-4 and a C10-18 alkyl group such as sodium polyoxyethylene lauryl sulfate; alkyl sulfosuccinic acid ester salts having a C8-18 alkyl group such as sodium laurylsulfosuccinate; and
natural surfactants, e.g., lecithin; glycerophospholipids; sphingophospholipids such as sphingomyelin; sucrose fatty acid esters of C12-18 fatty acids. Formulations of the present invention can contain one or more of these surfactants in combination. Preferred surfactants are polyoxyethylene sorbitan fatty acid esters such as Polysorbate 20, 40, 60 or 80, especially Polysorbates 20 and 80. Polyoxyethylene polyoxypropylene glycols such as poloxamers (e.g. Pluronic F-68®) are also preferred.

Sulfur-containing reducing agents include e.g., sulfhydryl-containing compounds such as N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and thioalkanoic acid having 1 to 7 carbon atoms.

Antioxidants include e.g., erythorbic acid, dibutylhydroxytoluene, butylhydroxyanisole, α-tocopherol, tocopherol acetate, L-ascorbic acid and salts thereof, L-ascorbyl palmitate, L-ascorbyl stearate, sodium bisulfite, sodium sulfite, triamyl gallate, propyl gallate or chelating agents such as disodium ethylenediamine tetraacetate (EDTA), sodium pyrophosphate and sodium metaphosphate.

Other components commonly added may also be contained, e.g., inorganic salts such as sodium chloride, potassium chloride, calcium chloride, sodium phosphate, potassium phosphate and sodium bicarbonate; and organic salts such as sodium citrate, potassium citrate and sodium acetate.

When the present invention is embodied as sustained release formulations, they can be prepared by known methods, e.g., by using a polymer as a base. Polymers used as bases are preferably biodegradable.

The therapeutic agents of the present invention are normally administered at a dose of 0.001 µg/kg/day to 1000 µg/kg/day, preferably 0.01 µg/kg/day to 100 µg/kg/day, more preferably 0.1 µg/kg/day to 30 µg/kg/day of an EPO derivative. The dosage of the therapeutic agents of the present invention to particular patients are determined by physicians in consideration of the age, weight, condition of the patients, the route of administration, etc.

In the therapeutic agents of the present invention, EPO derivatives are preferably topically administered. The site to which they are topically administered is not specifically limited, and they can be administered to any site at which angiogenesis should be promoted or blood flow should be increased (tissue, organ, etc.) or ischemic sites. Specific sites for topical administration include lower limb skeletal muscle, upper limb skeletal muscle, heart (heart muscle), etc.

Means for topical administration are not specifically limited so far as EPO derivatives can be efficiently administered to affected sites without significant systemic influence, and such means include conventional syringes, needles, topical needles, etc.

As used herein, topical administration normally means that an EPO derivative is administered directly to the site at which angiogenesis should be promoted or blood flow should be increased or an ischemic site, but it can also be administered to a vessel directly connected to the site at which angiogenesis should be promoted or blood flow should be increased or an ischemic site (e.g., an EPO derivative can be administered to the hepatic artery when the ischemic site is the liver, etc.).

As used herein, therapeutic agents also include prophylactic agents intended for prophylactic use.

### Stem cell-containing materials

Therapeutic effects of the therapeutic agents of the present invention increase when they are administered in combination with stem cell-containing materials.

Stem cells have the ability to self-replicate and to differentiate, and stem cells used in the present invention may be hematopoietic stem cells or other stem cells.

As used herein, stem cell-containing materials are not specifically limited so far as they are compositions containing stem cells, and they may comprise stem cells alone or other components in addition to stem cells. Stem cell-containing materials include, e.g., bone marrow, cord blood, peripheral blood, etc., and preferred stem cell-containing materials are bone marrow cells.

Bone marrow cells may be autogenous or exogenous. The histocompatibility antigen (HLA or MHC) type of the bone marrow administered may not be necessarily identical with that of the patient, but preferably identical. Bone marrow cells can be prepared by methods known to those skilled in the art, e.g., they can be collected from a part of the pelvis (ilium, femur, etc.) of a mammal under systemic anesthesia. Nucleated cell components or low gravity cell components may be separated from the collected bone marrow by known methods such as gravity centrifugation.

Bone marrow cells are typically administered at a dose of 1 x 10⁶ to 1 x 10¹² cells/body, preferably 1 x 10⁸ to 1 x 10¹⁰ cells/body, more preferably 3 x 10⁹ to 6 x 10⁹ cells/body.

The administration method is not specifically limited, and any method can be used, but topical administration is preferred, especially intramuscular injection to ischemic sites.

Bone marrow cells can be administered to multiple sites or at divided doses. For example, limb ischemia can be treated by intramuscularly administering 50 ml of a concentrated solution containing bone marrow cells to 50 sites (1 ml per site), or myocardial ischemia can be treated by intramuscularly administering 10 ml of a concentrated solution containing bone marrow cells to 50 sites (0.2 ml per site).

The present invention also provides blood flow enhancers, vascular promoters or therapeutic agents for ischemic diseases characterized in that an EPO derivative and a stem cell-containing material is combined. The EPO derivative and stem cell-containing material may be administered simultaneously or sequentially.

### Advantages of the Invention

As shown in the examples below, we performed an experiment on therapeutic effects using a hindlimb ischemia model, and observed that administration of asialoEPO alone more strongly inhibited hindlimb necrosis as compared with EPO alone (survival effect) and that administration of asialoEPO alone yielded recovery from cyanosis (substantial vascular regeneration effect) while administration of EPO alone did not yield recovery from cyanosis. Moreover, administration of asialoEPO in combination with bone marrow cell transplantation remarkably improved the survival effect and substantial vascular regeneration effect.

We also evaluated blood flow recovery effect by an experiment on therapeutic effects using the hindlimb ischemia model to find that asialoEPO was more effective for recovery of hindlimb blood flow than EPO whether it was administered alone or combined with bone marrow cell transplantation.

We also evaluated angiogenetic effect by an experiment on therapeutic effects using the hindlimb ischemia model to find that asialoEPO was more effective for angiogenesis than EPO whether it was administered alone or combined with bone marrow cell transplantation.

We also performed an experiment on the effects on the development of polycythemia using untreated ICR mice with no induced hindlimb ischemia to find that EPO administration resulted in enhanced erythroid hematopoiesis (Ret), enhanced extramedullary hematopoiesis in the spleen (Sp) and polycythemia (Hb, Hct) while administration of asialoEPO did not invite such adverse side effects.

Without wishing to be bound to the following theory, we presume that asialoEPO does not produce adverse side effects such as polycythemia because it degrades more rapidly than EPO in the blood and does not stay in the blood.

According to the present invention, EPO derivatives can be used for increasing blood flow, promoting angiogenesis or treating ischemic diseases without inviting undesirable effects such as polycythemia.

The following examples further illustrate the present invention without, however, limiting the same thereto. Various changes and modifications can be made by those skilled in the art, and these changes and modifications are also included in the present invention.

In the statistic analyses in the examples below, all data are represented by mean ± SD. Comparison between groups was performed by ANOVA, and then Fisher's exact test. Significant difference was defined as p<0.05.

### Examples

### Example 1: Preparation of asialoEPO

AsialoEPO was prepared by a method described in literature (Imai N. Higuchi M. Kawamura A. Tomonoh K. Oh-Eda M. Fujiwara M. Shimonaka Y. Ochi N. Physicochemical and biological characterization of asialoerythropoietin. Suppressive effects of sialic acid in the expression of biological activity of human erythropoietin in vitro. European Journal of Biochemistry. 194(2):457-62, 1990 Dec 12.) from EPO recombinantly prepared using CHO cells (Chugai Pharmaceutical Co., Ltd.). Removal of sialic acid was confirmed by molecular weight loss in SDS gel electrophoresis and pI shift in isoelectric focusing electrophoresis. Retention of biological activity was confirmed by a bioassay based on the growth of an EPO-dependent cell line.

### Preparation of asialoEPO

EPO freeze-dried powder and neuraminidase (Seikagaku Corporation) were dissolved in 10 mM citrate-phosphate buffer (pH 6.5) and mixed at 1 mg/ml and 250 mU, respectively. After incubation at 37°C for 1 hour, asialoEPO was purified by elution with acetonitrile concentration gradient on a C4 reverse-phase HPLC column (Vydac).

Removal of sialic acid from the resulting asialoEPO was confirmed by molecular weight loss in SDS gel electrophoresis and pI shift in isoelectric focusing electrophoresis. Specifically, 5.0 pg of asialoEPO was denatured at 100°C in SDS-PAGE sample buffer (TEFCO) for 10 minutes and electrophoresed in a 14 % acrylamide gel (TEFCO). The gel was stained with CBB to confirm molecular weight loss of about 5 kDa. After isoelectric focusing electrophoresis on PhastGel IEF 3-9 (Amersham), the sample was fixed with a 20% trichloroacetic acid solution and stained with silver using PhastGel Protein Silver Staining Kit. It was found that pI shifted to around 8.0 by dissociation of sialic acid.

### Biological activity of asialoEPO

Retention of biological activity in asialoEPO was confirmed by a bioassay based on the growth of an EPO-dependent cell line. Specifically, 10000 cells of the EPO-dependently growing cell line AS-E2 (reference: Miyazaki Y. Kuriyama K. Higuchi M. Tsushima H. Sohda H. Imai N. Saito M. Kondo T. Tomonaga M. Establishment and characterization of a new erythropoietin-dependent acute myeloid leukemia cell line, AS-E2. Leukemia. 11(11):1941-9, 1997 Nov.) were cultured in a 20% FCS/IMDM medium (Hyclone) containing 0.04 to 10 ng/ml of asialoEPO or EPO at 37°C, under 5.0% CO₂ for 3 to 4 days, and the number of surviving cells was measured with WST-1 reagent (Takara Shuzo). It was found that asialoEPO also stimulates growth in the same manner as EPO.

### Example 2: Preparation of a hindlimb ischemia model of ICR mice

Eight-week old male ICR mice (30 - 35g) were purchased from Charles River (Yokohama, Japan) and used for the experiment. All the experimental procedures were aseptically performed according to Guide for the Care and Use of Laboratory Animals (NIH publication No.86 - 23; National Institute of Health, Bethesda, MD). The mice were anesthetized by intraperitoneal administration of ketamine (60 mg/kg BW) and xylazine (6 mg/kg BW). The skin in the midpoint of the left hindlimb was incised by the method of Isner to expose vessels (Couffinhal T, Silver M, Zheng LP, Kearney M, Witzenbichler B, Isner JM: Mouse model of angiogenesis. Am J Pathol 152: 1667 - 1679, 1998). The femoral artery was ligated at its origin and then its distal saphenous artery was ligated, and the other side branches were separated and excised with the main artery.

All the mice had access to ordinary diet and drinking water.

### Example 3: Experiment on therapeutic effects using the hindlimb ischemia model (survival effect and vascular regeneration effect)

### (Experimental method)

A therapeutic experiment was performed using the hindlimb ischemia model of ICR mice prepared by the method of Example 2. In the experiment, hindlimb necrosis was defined as death (survival). Recovery of hindlimb from cyanosis was defined as recovery (recovery).

The following groups were used in the experiment (n=13 in each group).
Group C: untreated.
Group E: treated with EPO (400 U/kg body weight, via intramuscular injection to ischemic sites) for consecutive 6 days.
Group AE: treated with asialoEPO at the same dose by the same administration method.
Group B: treated with 1 X 10⁷ bone marrow cells via intramuscular injection to ischemic sites (4 sites on day 1 of administration).
Group B+ E: combination of B (bone marrow cells) and E (EPO).
Group B+ AE: combination of B (bone marrow cells) and AE (asialoEPO).

### (Results)

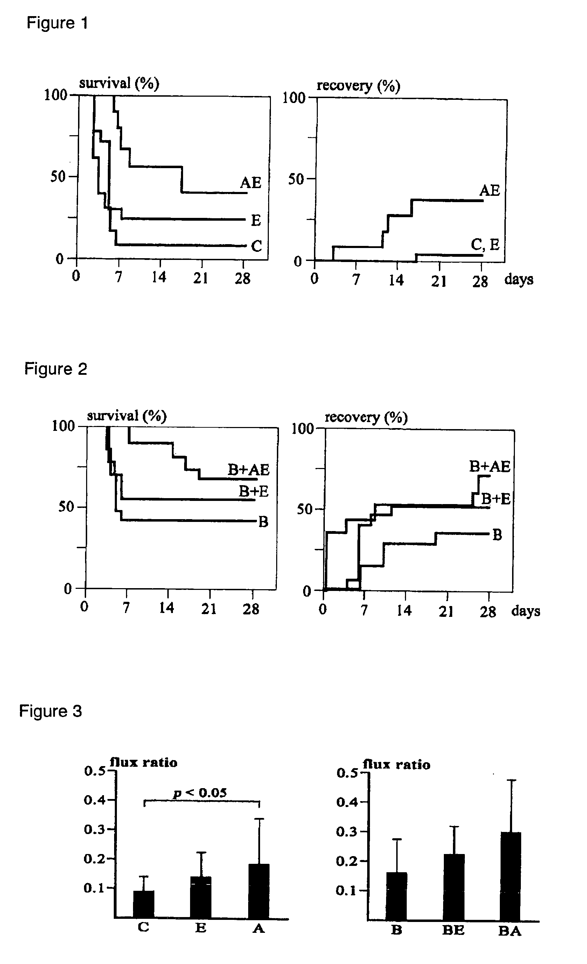

The influences of each therapy on survival effect and recovery effect are shown in Figure 1 and Figure 2. The results are summarized as follows.
(1) Administration of EPO alone inhibits hindlimb necrosis, but administration of asialoEPO alone more strongly inhibits hindlimb necrosis (survival effect).
(2) Administration of EPO alone does not yield recovery from cyanosis, but administration of asialoEPO alone yields recovery from cyanosis (substantial vascular regeneration effect).
(3) Survival effect and substantial vascular regeneration effect are observed by bone marrow cell transplantation, but these effects are promoted by combining it with EPO administration, and these effects are further remarkably promoted by combining it with administration of asialoEPO in place of EPO.

### Example 4: Experiment on therapeutic effects using the hindlimb ischemia model (blood flow recovery effect)

Blood flow was evaluated using a mouse hindlimb ischemia model prepared from the same mice by the same method as used for the hindlimb survival/recovery model in Example 3.

### (Experimental method)

Therapy was started on the day the hindlimb ischemia model was prepared (day 1). Transplantation was performed on day 1. Administration of EPO or the like was performed on days 1 - 6 for 6 days. On day 7, recovery of hindlimb blood flow was assessed by laser Doppler flowmetry.

The following groups were used in the experiment.
Group C: intramuscular injection of physiological saline for 6 days (n=12)
Group E: intramuscular injection of EPO at 400 IU/kg body weight for 6 days (n=5)
Group A: intramuscular injection of asialoEPO at 400 IU/kg body weight for 6 days (n=10)
Group B: transplantation of allogeneic and syngeneic bone marrow cells (1x10⁷ bone marrow cells) (n=7)
Group BE: combination of B and E (n=5)
Group BA: combination of B and A (n=6)
Blood flow in both hindlimbs was measured using a moorLDI laser Doppler system from Moor Instruments (Wilmington, Delaware, USA), and the value measured in the ischemic limb was divided by the value measured in the healthy limb to determine the flux ratio.

### (Results)

The results are shown in Figure 3. AsialoEPO was more effective for recovery of hindlimb blood flow than EPO whether it was administered alone or combined with bone marrow cell transplantation.

### Example 5: Experiment on therapeutic effects using the hindlimb ischemia model (angiogenetic effect)

### (Experimental method)

After blood flow was measured by Laser Doppler flowmetry in Example 4, the ischemic hindlimb femoral muscle was removed to prepare a specimen, and blood vessels were stained with an anti-CD31 antibody and peroxidase. The number of vessels and the number of muscle fibers were counted, and the former was divided by the latter to determine the number of vessels per muscle fiber.

### (Results)

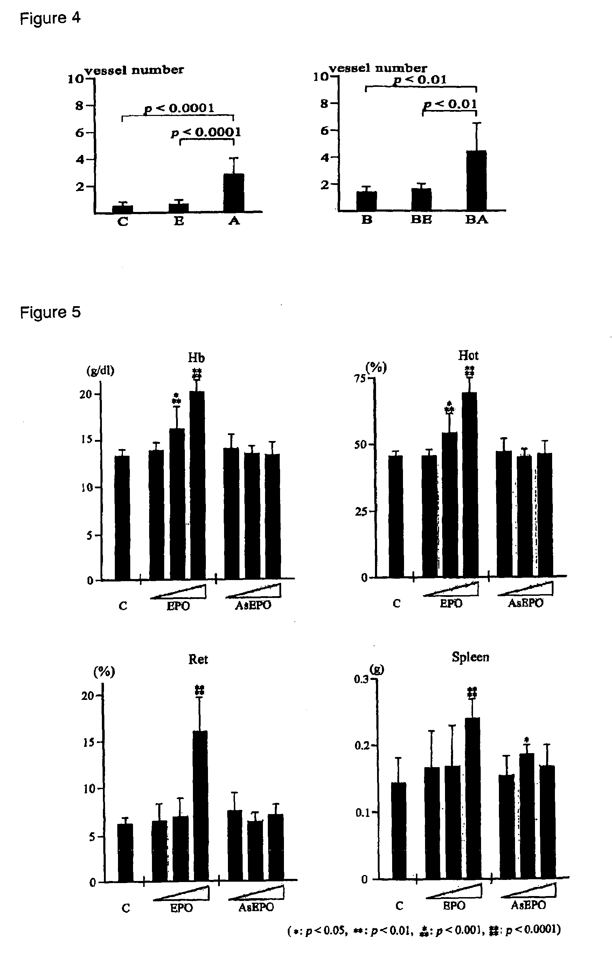

The results are shown in Figure 4. AsialoEPO was more effective for angiogenesis than EPO whether it was administered alone or combined with bone marrow cell transplantation.

### Example 6: Effects on the development of polycythemia

### (Experimental method)

For the purpose of observing the development of polycythemia among adverse side effects of EPO, untreated ICR mice with no induced hindlimb ischemia were treated with EPO or asialoEPO for consecutive 6 days (intramuscular injection to the hindlimb), and blood was collected 10 days after the first administration and then euthanized, and the spleen (Sp) was weighed. Hemoglobin concentration (Hb), hematocrit value (Hct) and reticulocyte ratio (Ret) were determined.

The following groups were used in the experiment (n=8 in each group).
Group C: control
Group E: treated with EPO at a dose of 40, 400 or 4,000 U/kg body weight
Group AE: treated with asialoEPO at the same dose.

### (Results)

The effects of each therapy on Sp: spleen weight, Hb: Hemoglobin concentration, Hct: hematocrit value, and Ret: reticulocyte ratio are shown in Figure 5. The results are summarized as follows.
(1) Administration of EPO resulted in enhanced erythroid hematopoeisis (Ret), enhanced extramedullary hematopoiesis in the spleen (Sp) and polycythemia (Hb, Hct).
(2) Administration of asialoEPO did not invite such adverse side effects.

## Claims

1. A therapeutic agent for ischemic diseases containing an erythropoietin (EPO) derivative as an active ingredient.

2. The therapeutic agent of claim 1 wherein the EPO **derivative is asialoEPO.**

3. The therapeutic agent of claim 1 or 2, which is administered in combination with a stem cell-containing material.

4. The therapeutic agent of claim 3 wherein the stem cell-containing material is bone marrow cell.

5. A vascular promoter containing an EPO derivative as an active ingredient.

6. The promoter of claim 5 wherein the EPO derivative is asialoEPO.

7. The promoter of claim 5 or 6, which is administered in combination with a stem cell-containing material.

8. The promoter of claim 7 wherein the stem cell-containing material is bone marrow cell.

9. A blood flow enhancer containing an EPO derivative as an active ingredient.

10. The enhancer of claim 9 wherein the EPO derivative is asialoEPO.

11. The enhancer of claim 9 or 10, which is administered in combination with a stem cell-containing material.

12. The enhancer of claim 11 wherein the stem cell-containing material is bone marrow cell.
